# EUROPEAN PATENT SPECIFICATION

(11) **EP 1 985 579 B1**
(45) Date of publication and mention of the grant of the patent: **10.01.2018**
(21) Application number: 08154893.5
(22) Date of filing: 21.04.2008
(51) Int. Cl.: B81C 3/00, A61N 1/05, A61B 5/04, A61B 5/0478

(54) **Connecting scheme for the orthogonal assembly of microstructures**
Schaltdiagramm für die orthogonale Anordnung von Mikrostrukturen
Schéma de connexion pour l'assemblage orthogonal de microstructures

(30) Priority: 27.04.2007 US 926642 P
(43) Date of publication of application: 29.10.2008
(73) Proprietor: IMEC VZW, 3001 Leuven (BE); Katholieke Universiteit Leuven, 3000 Leuven (BE); Albert-Ludwigs-Universität Freiburg, 79085 Freiburg (DE)
(72) Inventor: Aarts, Arno, 3000 Leuven (BE); Neves, Hercules Pereira, 1320 Hamme-Mille (BE); Van Hoof, Chris, 3001 Leuven (BE); Beyne, Eric, 3001 Leuven (BE); Puers, Robert, 3052 Blanden (BE); Ruther, Patrick, 76189 Karlsruhe (DE)
(74) Representative: Awapatent AB

(56) References cited:
- US-A- 4 461 304
- US-A1- 2007 087 474
- HOOGERWERF A C ET AL: "A three-dimensional neural recording array" TRANSDUCERS. SAN FRANCISCO, JUNE 24 - 27, 1991; [PROCEEDINGS OF THE INTERNATIONAL CONFERENCE ON SOLID STATE SENSORS AND ACTUATORS], NEW YORK, IEEE, US, vol. CONF. 6, 24 June 1991 (1991-06-24), pages 120-123, XP010037223 ISBN: 978-0-87942-585-2
- WISE K D: "Silicon microsystems for neuroscience and neural prostheses" IEEE ENGINEERING IN MEDICINE AND BIOLOGY MAGAZINE, IEEE SERVICE CENTER, PISACATAWAY, NJ, US, vol. 24, no. 6, 1 September 2005 (2005-09-01), pages 22-29, XP001512612 ISSN: 0739-5175
- HETKE J. F. ET AL.: "3-D silicon probe array with hybrid polymer interconnect for chronic cortical recording" PROCEEDINGS OF THE 1ST INTERNATIONAL IEE EMBS CONFERENCE ON NEURAL ENGINEERING 2003, 20 March 2003 (2003-03-20), - 22 March 2003 (2003-03-22) pages 181-184, XP002538007

## Description

### Technical field of the invention

The invention relates to the field of microsystem integration. More particularly, the present invention relates to a device for sensing and/or actuating and a method for assembling probes for sensing and/or actuating on a substrate.

### Background of the invention

In-plane to in-plane multi-contact MEMS connectors have been described by M.P. Larsson et al. (in IEEE J. Microelectromech. Sys., vol. 13, no. 2, pp. 365-376, 2004) and by T. Akiyama et al. (in Proc. 2001 Intl. Microprocesses & Nanotech. Conf., Shimane (Japan), pp. 52-53, 2001). In both cases, it is not possible to achieve in-plane to off-plane multi-contact connection.
M.P. Larsson et al. describe overhanging blades to provide spring action. As there is no bending of these structures into a cavity, there is no possibility of using this technology to assemble an orthogonal off-plane device onto this connector. Also multi-contact connection is not possible.

Toshiyoshi et al. (in Proc. SPIE, vol. 3680, pp. 679-686, 1999) describe the fabrication of fingers and matching holes; the fingers have metal plating on one of their surfaces and the hole has metal around it. Malhi et al., in US5031072, describe bonding at the corner between a motherboard and an upright connecting part. In both cases, multi-contact connection is not possible.

A three-dimensional probe array for neural studies assembling combs of probes onto a backbone was obtained by fusing single contacts together through plating (A.C. Hoogerwerf et al. in IEEE Trans. Biomed. Eng., vol. 41, no. 12, pp. 1136-1146, 1994) or through corner bonding (Q. Bai et al in IEEE Trans. Biomed. Eng., vol. 47, no. 3, pp. 281-289, 2000). These connecting structures (cavities and matching posts) cannot contain a plurality of contacts. This approach results in a 3D probe array which requires additional space beyond the implanted length of the probe and thus increases the overall thickness of the platform.

US2004082875 describes a modular approach to building microprobe arrays for the brain that can incorporate probes of different pitches and different lengths. Each probe is made of conductive material and can only have one electrode.

US5215088 describes a fixed array of needles made out of a single piece of silicon. This approach is not modular and has no interconnect scheme.

US2006108678 describes an electroplating-based technology for building probe arrays. The approach is not modular and each probe can only have one electrode.

EP1637019 describes an interconnect scheme that permits connecting a two-dimensional array of spring structures to the land grid array.

N. Tanaka and Y. Yoshimura (Electronic Components and Technology Conference 2006, p814 - 818) presented stacked dies using conventional in-plane flip chip technique in which the connection is made by using the caulking technique at room temperature. It is not possible to achieve in-plane to off-plane multi-contact connection.

US6829498 presents an implant device for neural interface with the central nervous system. The device may be configured as a three-dimensional structure and is capable of sensing multi-unit neural activity. The device has a big base that increases the overall thickness of the platform to be inserted in the skull. US 2007/087474 A1 discloses a method of assembling a three dimensional micromachined structure comprising the steps of defining a cavity in a holder wafer having a thick upper layer, providing a plurality of fingers in the thick upper layer extending from the holder wafer into the cavity, and disposing an out-of-plane wafer into the cavity in the holder wafer in engagement with the fingers to hold the out-of-plane wafer in place in an out-of-plane position with respect to the holder wafer.

A. C. Hoogerwerf et al. (XP010037223) describes a three-dimensional recording array for the high-density monitoring of neural activity throughout a volume of cortical tissue. The microassembly techniques used permit multiple multishank planar probes to be precisely configured to form 3D microstructures with probe spacings of 100 micrometres or less. The probes are inserted through slots in an orthogonal platform, also formed by silicon micromachining.

### Summary

It is an object of embodiments of the invention to provide a device for sensing and/or actuating and a method for assembling, on a substrate, probes for sensing and/or actuating.

The above objective is accomplished by a method and device according to the present invention.

In a device and method according to embodiments of the invention, microstructures, also referred to as probes, with different functionality and dimensions can be orthogonally assembled in a modular way in a thin or slim base backbone with multiple interconnects.

The present invention relates to a device and method as disclosed in the appended claims.

In a first aspect, the present invention provides a device for sensing and/or actuating as defined in the appended independent claim 1, in particular for sensing and/or actuating neural activity.

The dimensions of the at least one cavity are substantially matching the dimensions of the connector part of the at least one microstructure.

The at least one cavity and the connector part may have dimensions between 50 µm and 2000 µm.

The angle between the substrate and said at least one microstructure may be between 45° and 90°.

The width of the blades and the length of the second part of the blade may be between 1 µm and 100 µm.

According to embodiments of the invention, the device may furthermore comprise conductive paths on the substrate connecting the conductive blades with bond pads and/or integrated circuitry in the substrate.

The device may furthermore comprise functional areas on the at least one microstructure in contact with the at least one conductive area.

The device may furthermore comprise first microfluidic channels in the substrate and second microfluidic channels in the at least one microstructure, the first and second microfluidic channels being connected to each other with sealed holes in the cavities.

According to embodiments of the invention, the at least one microstructure may be a needle.

The substrate may have a thickness between 200 µm and 2000 µm.

According to embodiments of the invention, the substrate may be a semiconductor, e.g. silicon wafer or a thinned semiconductor, e.g. silicon wafer covered with insulating material, e.g. silicon oxide.

The conductive areas and the blades comprise at least one conductive material.

The blades comprise a flexible material.

The substrate, the at least one microstructure, the at least one conductive blade and the at least one conductive area may be made of or covered with biocompatible materials.

In a further aspect, the present invention provides the use of a device according to embodiments of the present invention for measurements and/or actuation of neural activity.

In another aspect, the present invention provides a method for assembling on a substrate microstructures for sensing and/or actuating as defined in the appended independent claim 11, in particular for sensing and/or actuating neural activity.

The method may furthermore comprise fabricating functional areas on the microstructures in contact with the at least one conductive area.

The method may furthermore comprise providing bond pads on the substrate and/or integrated circuitry in the substrate and providing conductive paths on the substrate connecting the at least one flexible conductive blade at one side and the bond pads on the substrate and/or integrated circuitry in the substrate at the other side.

According to embodiments of the invention, the method may furthermore comprise:
- providing first microfluidic channels in the substrate and second microfluidic channels in the microstructures, whereby each of the first microfluidic channels is connected to at least one of the second microfluidic channels via a hole in the at least one cavity; and
- sealing the holes in the at least one cavity.

The method may furthermore comprise connecting the at least one flexible conductive blade and/or the microfluidic channels to measurement equipment.

Providing the at least one flexible conductive blade may comprise:
- filling the at least one cavity with a sacrificial material;
- providing at least one conductive blade partially on the sacrificial material;
- optionally providing conductive paths in electrical contact with the at least one blade; and
- removing the sacrificial material from the cavities.

The sacrificial material may, for example, be polyimide or Benzocyclobutene (BCB).

Providing the at least one flexible conductive blade and the conductive paths may be performed by metal deposition and lift-off or by metal deposition and patterning by dry and/or wet etching.

Realizing electrical contact between the at least one flexible conductive blade and the at least one conductive area may be done by caulking.

The dimensions of the connector part may be slightly larger or smaller than the dimensions of the cavities. In that case, fixing the connector parts in the cavities may comprise:
- creating a temperature difference between the substrate and the microstructure such as to allow insertion of the connector parts into the cavities;
- inserting the connector parts in the cavities; and
- bringing the substrate and the microstructure at the same temperature.

In another aspect, the present invention provides a device for connecting microstructures and/or probes with different functionalities to other equipment, each microstructure and/or probe having a connector part. The device, as defined in the appended independent claim 1, comprises
- an insulating substrate;
- cavities in said insulating substrate, the dimensions of said cavities being chosen such that a connector part of a microstructure and/or probe can be inserted in said cavities. The dimensions of said cavities are chosen to match the dimensions of the connector part of the microstructure and/or probe; and
- flexible blades overhanging said cavities.

According to the present invention, the flexible blades are conductive. The conductive flexible blades are adapted for matching conductive strips or regions on said connector part of said microstructures and/or probes. In this case conductive strips on the connector part of the microstructure and/or probe can be electrically connected to functional regions on the microstructure and/or probe. These functional regions on the microstructure and/or probe allow different measurements and can also allow activation. When the microstructure and/or probe is inserted in a cavity, the overhanging conductive blades or flaps bend inside the cavity and contact the corresponding conductive strips or regions on the connector part of the microstructure and/or probe.

A device according to embodiments of the present invention may further comprise conductive lines on said insulating substrate in between said cavities, whereby said conductive lines are at one side in electrical contact with said conductive flexible blades or flaps and at the other side in contact with integrated circuitry or bond pads or conductive strips on said substrate.

A device according to embodiments of the present invention may further comprise first microfluidic channels in said insulating substrate and second microfluidic channels in at least one of said microstructures and/or probes. One end of said first microfluidic channels is located in the cavity or cavities. One end of said second microfluidic channels is located at the connector part of the microstructure and/or probes. Each of said one ends of said first microfluidic channels is adapted in shape and location to be in contact with a corresponding one of said one ends of said second microfluidic channels.

In yet another aspect, the present invention provides a method for fabricating a device for connecting microstructures and/or probes with different functionalities to other equipment. Each microstructure and/or probe has a connector part. The method comprises the steps of
- providing a semiconductor, e.g. silicon, wafer;
- etching cavities in said semiconductor, e.g. silicon, wafer,
- depositing insulating material, e.g. silicon dioxide, on said semiconductor, e.g. silicon, wafer;
- coating said substrate at the side of the cavities with a sacrificial material, for example polyimide;
- planarizing said wafer surface thereby completely removing the sacrificial material, e.g. polyimide layer, from the non-etched regions of the wafer;
- depositing conductive, e.g. metal, blades or flaps partially on the sacrificial material, e.g. polyimide, on said cavities;
- depositing conductive, e.g. metal, tracks in electrical contact with said blades or flaps; and
- removing the remaining sacrificial material, e.g. polyimide, from the cavities.

In yet another aspect, the present invention provides a method for fabricating a device for connecting microstructures and/or probes with different functionalities to other equipment. Each microstructure and/or probe has a connector part. The method comprises the steps of
- providing a semiconductor, e.g. silicon, wafer;
- etching cavities in said semiconductor, e.g. silicon, wafer,
- depositing insulating material, e.g. silicon dioxide, on said semiconductor, e.g. silicon, wafer;
- coating said substrate at the side of the cavities with a sacrificial material, for example polyimide;
- planarizing said wafer surface thereby leaving a layer of sacrificial material, e.g. polyimide, on the wafer;
- removing the sacrificial material, e.g. polyimide, on at least the non-etched regions. This means that all sacrificial material, e.g. polyimide, is removed where no cavities are present. At the edges of the cavities also some sacrificial material, e.g. polyimide, can be removed. The sacrificial material, e.g. polyimide, on top of the major part of the cavities is not etched. At these locations on the cavities the sacrificial material, e.g. polyimide layer, is located above/at a higher level than the initial substrate surface;
- depositing conductive, e.g. metal, blades or flaps partially on the sacrificial material, e.g. polyimide, on said cavities;
- depositing conductive, e.g. metal, tracks in electrical contact with said blades or flaps; and
- removing the remaining sacrificial material, e.g. polyimide, from the cavities.

In a method according to embodiments of the present invention, providing cavities in said semiconductor, e.g. silicon, substrate may comprise the steps of
- coating said semiconductor, e.g. silicon, wafer with a layer of insulating material, e.g. silicon dioxide;
- patterning said insulating material, e.g. silicon dioxide. This can be done by lithography, followed by etching of the insulating material, e.g. silicon dioxide layer, using any suitable etching process, e.g. reactive ion etching (RIE); and
- transferring said patterns in said insulating material, e.g. silicon dioxide layer, to the underlying semiconductor material, e.g. silicon. This can be done by any suitable method, e.g. deep reactive ion etch (DRIE).

In a method according to embodiments of the present invention, depositing said conductive, e.g. metal, blades and said conductive, e.g. metal, tracks in electrical contact with said blades may comprise the steps of
- depositing a conductive, e.g. metal, seed layer on the entire surface;
- defining patterns for the conductive, e.g. metal, tracks and overhanging blades or flaps in a resist layer;
- electroplating conductive material, e.g. gold. This will be plated on the regions not protected by the resist;
- removing said resist layer; and
- removing the seed layer in the areas not covered with the conductive material, e.g. gold.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1: Sketch of a backbone concept wherein combs or probes are assembled to a 3D array.
Fig. 2: Cross-sectional sketch of the assembling process. (a) The female connecting microstructure contains a series of overhanging conductive blades (lines). (b) The male connecting microstructure contains matching conductive strips (lines top part). (c) As insertion takes place, the male connecting microstructure pushes the conductive blades into the cavity of the female connecting microstructure. (d) The conductive blades end up squeezed between the side wall of the female connecting microstructure cavity and the male connecting microstructure.
Fig. 3: (a) cross-sectional side view of a device illustrating clamping of a connector part of a probe in a cavity in a base by means of a conducting blade. (b) and (c) are respectively a top view and a three-dimensional view of a structure for clamping a probe in cavities in a base.

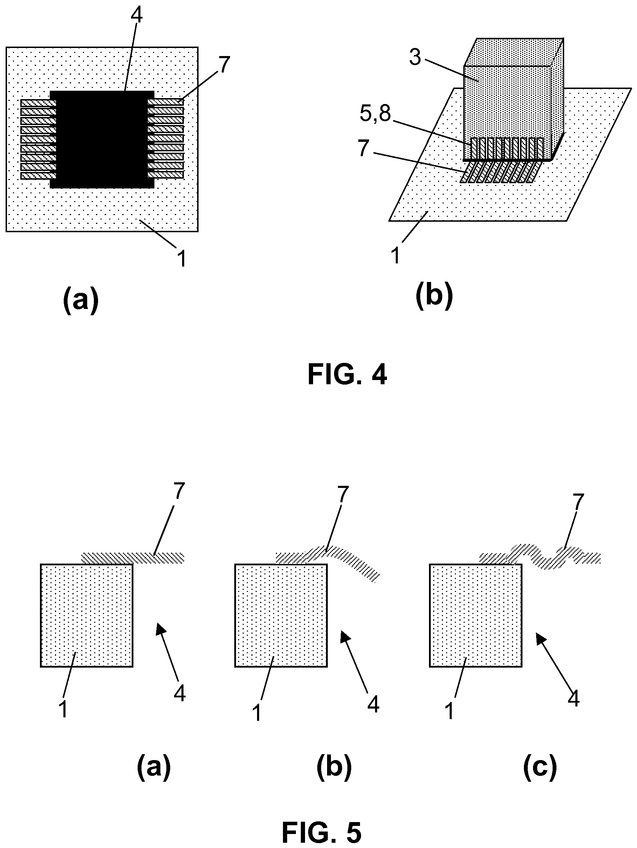

Fig. 4: (a) Top view of the cavity showing a plurality of contacts or overhanging blades within one microstructure. The dark rectangle shows the cavity itself and the conductive blades leading over the edge of the cavity are in dashed lines. (b) A male connecting microstructure (dotted at the top) is inserted into the female connecting microstructure (dotted at the bottom). It shows that in-line metal tracks (dashed lines) on the microstructures match metal tracks at the cavity edge. The contact is established by contact blades, leading over the edge of the cavity bending into the cavity and being squeezed between the cavity wall and the contact pad of the inserted microstructure.
Fig. 5: Examples of geometry of the blades. The intention of the different profiles is to relieve the stress imposed on the blade during insertion of the male connecting microstructure and/or to provide extra spring action to push the blade against the matching conductive strip.
Fig. 6: Cross-section of an assembled male-female pair, including electrodes and microfluidic channels in/on both connecting microstructures. Once assembled, a single microfluidic conduit is created through the male and female parts.
Fig. 7: Close-up of details of the cavity, including electrical wiring towards the blades. The cavity is depicted in dashed lines and the conductive material, e.g. metal, (which contains all the connecting tracks and as well as the overhanging metal blades) is depicted in black.
Fig. 8: Design of electrical connections on the base, containing a 4x4 array of cavities.

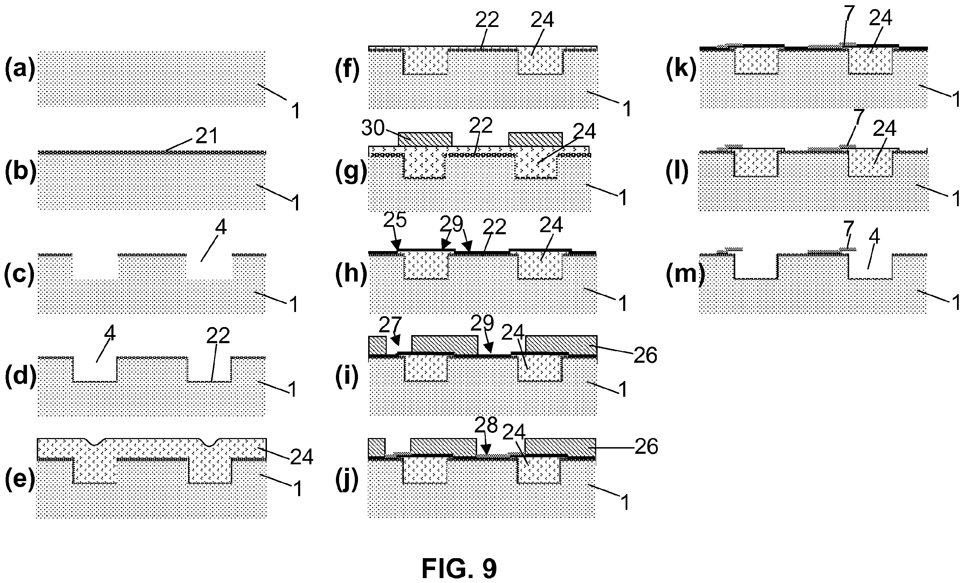

Fig. 9: Fabrication steps. The process starts (a) with a semiconductor material, e.g. silicon wafer, which is coated (b) with a layer of electrically insulating material, e.g. plasma-enhanced vapor deposition (PECVD) of silicon dioxide. Lithography is performed, followed by the etching of the electrically insulating material, e.g. oxide layer, using any suitable method, e.g. reactive ion etching (RIE) and the transfer of the patterns formed by lithography to the underlying semiconductor material, e.g. silicon, by any suitable method, e.g. deep reactive ion etch (DRIE), (c). Another layer of electrically insulating material, e.g. PECVD silicon dioxide, is provided (d) e.g. deposited to provide electrical insulation inside the cavities, followed by the coating (e) of the surface with a thick layer of sacrificial material, e.g. polyimide. An initial planarization step (f) is performed e.g. by grinding the sacrificial material, e.g. polyimide, with a wafer grinder, leaving a layer of polyimide on the top regions of the wafer. A second lithography step is performed to create a step on the sacrificial material, e.g. polyimide, which will add spring action to the blades and facilitate their bending. This is followed by RIE of the polyimide to completely remove it from the top regions around the lithographically defined areas (g). A conductive, e.g. metal, seed layer is deposited (h) on the entire surface. A resist layer is provided, and a third lithography step is done (i) to define the patterns for the metal (tracks and overhanging blades). Conductive material, e.g. gold, is then electroplated (j) on the regions not protected by the resist, after which the resist is removed (k). The seed layer is etched from the open areas (I) and the remaining sacrificial material, e.g. polyimide, is completely removed from the cavities (m). As a result the blades are hanging over the cavity.
Fig. 10: Cross-section of (a) a blade and (b) the shape of a blade resulting from the use of a lithographically defined step around the cavity (as obtained using the process described in Fig. 9).
Fig. 11: Scanning electron micrograph of overhanging blades on a cavity.
Fig. 12: Scanning electron micrograph showing the two step electroplated gold clips leading over the edge of the cavity.
Fig. 13: Scanning electron micrograph of a 4x4 probe array on a base backbone.
Fig. 14: Schematic illustration of (a) microsystems connected in an in-plane fashion and (b) microsystems connected in an out-of-plane fashion, also called orthogonal assembly of microsystems.

In the different figures, the same reference signs refer to the same or analogous elements.

### Detailed description

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and that the embodiments of the invention described herein can operate in other orientations than described or illustrated herein.

The term "comprising", used in the claims, should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

Understanding of neuronal operations in the brain can be achieved by electrical recording of single neuron activity. Recordings from the brain allow investigating the activity of individual neurons, especially the interaction between electrical and chemical signals related to short and long term changes of morphology and information transfer. These measurements are often performed with single electrodes or small ensembles of electrodes.

Multi-electrode arrays can allow simultaneous recording of electrical signals at different locations. This is advantageous as this can increase the number of neurons recorded and can give information on the interaction between different neurons. Often multiprobe arrays only allow sampling in a given plane, either a plane parallel to the surface or a plane orthogonal to the surface.

Arrays in which 3D geometry can be achieved, would allow 3D recordings of parts of the brain. This can provide laminar information and information on the functional organization such as columnar or patchy organizations in primary and associative cortex. That way it could become possible to record the local network level between neurons, i.e. the interaction of neighbouring neurons can be recorded. If stable recordings from the same cortical regions can be obtained over extended periods of time, changes in population activity can be studied, both at single neuron level and at the interaction level with learning, memory and training.

Multi-electrode arrays of which the 3D geometry can be adapted to the folding of the cortex can be interesting. This can be achieved with a modular approach, allowing the individual assembly of multiple probes with customized architecture into three-dimensional arrays to address specific brain regions. That way, sulci of highly folded cortices such as those of humans can be contacted.

Also interesting are correlation studies in which the causal relationship between neuronal stimulation or inactivation and neuronal activity is investigated. Neuronal stimulation can be achieved by electrical stimulation techniques using for example microwires. Inactivation can for example be achieved by injecting locally pharmacologically active substances to temporally silence neuronal activity. The location of stimulation or inactivation needs to be assessed functionally. Therefore multifunctional arrays can be used in which recording can be combined with stimulation and fluid can be delivered through the recording channels. Integration of electrical recording with direct measurement of transmitter release can allow describing the functioning of a cerebral region both in electrical and chemical terms,

A modular approach allows the integration of recording and stimulation electrodes, biosensors, microfluidics and integrated electronics. When the individual probes are present, a device can quickly be assembled according to the particular application.

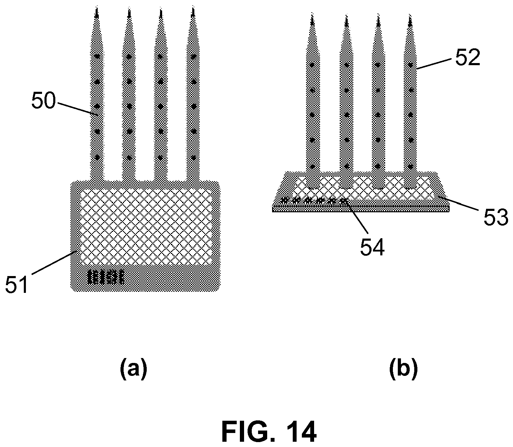

Microfabricated elements are often fabricated in an in-plane fashion (see Fig. 14(a)) and electrical connections in microsystems are often having in-line connectivity, i.e. between microsystems 50 that are parallel to each other. In that case, a substrate 51 is processed additively, for example by surface micromachining through the deposition, patterning and etching of thin films or subtractively, for example by bulk micromachining through the patterning and etching of the substrate 51 or a combination of both. The resulting structure is then predominantly in the same plane as the original substrate 51.

Orthogonal assembly of a microsystem 52 onto a substrate 53 (Fig. 14(b)) uses in-line connections using the edge of the standing piece or bonding to metal strips one by one to establish connection between each pair of orthogonal contact pads 54. This assembling method tends to be time consuming, expensive, and unreliable.

In the present invention, in-plane structures are assembled in an out-of-plane fashion, i.e. structures are assembled orthogonally on a common base. The structures can also make a non-orthogonal angle to the base. That way spatial complexity is enabled while preserving functionality such as electrical and fluidic interconnects. Matching mechanical and electrical features of two microstructures are orthogonally joined together or under a predetermined angle. This invention consists in a connector mechanism that makes it possible to assemble microfabricated elements (i.e. microstructure/probe and base) orthogonally or under an angle, to make a microsystem.

An advantage of embodiments of the present invention is the fact that the base or backbone can be made thin, e.g. thinner than found in prior-art. This can be interesting for several applications, as the base consumes only limited additional space beyond the implanted length of the probe array. For example in neural applications this can be interesting. In that case it may allow the base with probes to float with the brain (i.e. to move together with the brain, so not to be attached to the skull).

In the text below, the base, backbone, or support all mean the same connecting device. In the base, there are cavities, also called holes. Overhanging these cavities, there are blades, also called flaps or clips. In the base, microstructures or probes can be inserted.

In embodiments of the present invention a modular approach for assembling probes is presented. One or more of the following features are provided in any or all of the embodiments of the present invention:
- Possibility to assemble probes of multiple lengths and configurations (electrode quantity and pitch, presence of open areas, multiple probe profiles, etc), which allows probing in 3 dimensions.
- Possibility to assemble probes orthogonally or under an angle different from 180° on the base.
- Possibility to assemble probes of different functionalities (electrical measurements and stimulation, temperature sensing, drug delivery, biosensors) combined with electrical probes for recording and stimulation, in any desired configuration and size.
- Electrical connection between the conductors on the probe and the conductors on the base can be achieved orthogonally.
- Possibility to include various/more than one electrical connection on one probe.
- Possibility to include different functionalities on one probe.
- Microfluidic channels can be introduced, thereby allowing integration with fluidic valves and pumps.
- Electrical connection with integrated electronic circuitry on the base or electrical connection with other equipment (for measurement and analysis) is possible.
- Possibility to align, mechanically lock or clamp probes in the base.
- The possibility to use a very thin base.
- Scalability.

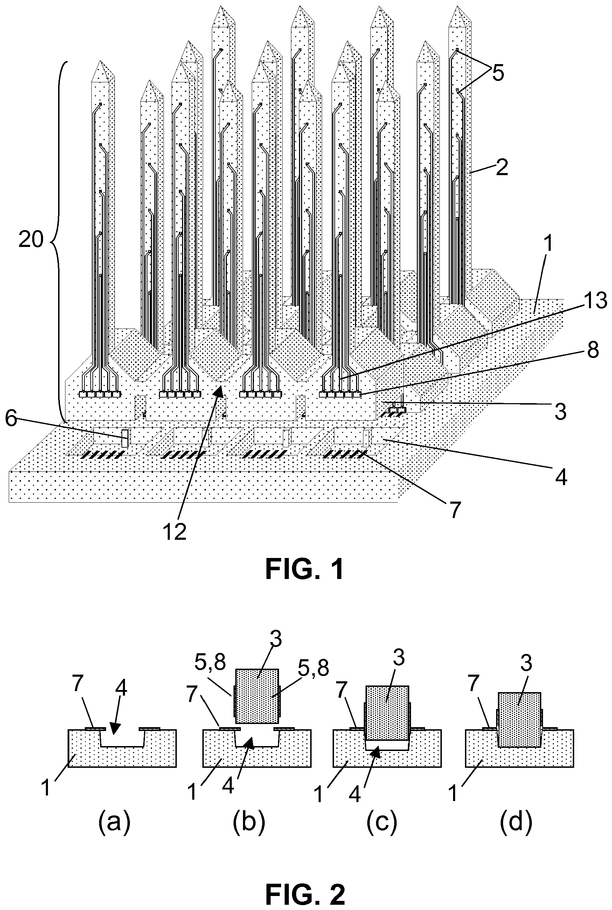

The actual invention (see Fig. 1) provides a device for sensing and/or actuating comprising a substrate 1, e.g. a semiconductor substrate such as a silicon substrate, also referred to as base or support or backbone, in which microstructures 20, also referred to as probes or out-of-plane structures, with different functionality and dimensions can be inserted in a modular way. In the following description the terms substrate, base, support and backbone will be used next to each other. It has to be understood that these terms all indicate the same thing, i.e. that the female part of the device in which cavities are formed for providing the connector parts for the microstructures 20. Similarly, the terms microstructures, probes or out-of-plane structures will also be used next to each other and are also intended to indicate a same entity, i.e. the male structures that are inserted in cavities 4 of the substrate 1 for forming the device according to embodiments of the invention. The microstructures 20 comprise a shaft 2 and a connector part 3, the connector part 3 having a shape matching in cavities 4 in the base 1. Electrical interconnection of the electrodes to an external circuitry as well as connections to microfluidic channels can be obtained via the common base 1 (see further). That way multiple microstructures 20 can be integrated to a 3D-probe array. The base 1 can provide mechanical support, electrical connectivity, and connectivity between microfluidic channels between the probes 20 and the outside world. The base 1 can be made thin, depending on the depth of the cavities 4 (see further).

An example of an application of the device according to embodiments of the present invention provides a base 1, also called support or backbone, in which probes 20 for cerebral applications with different functionality and dimensions can be inserted in a modular way. In particular, a combination of electrodes 5 on a probe 20 that could be a needle-like structure having electrodes 5 at the tip and along the shaft 2 can be used. At the bottom a broader connector part 3 can be provided to be inserted in the base 1 (see Fig. 1). The modular approach allows assembling the probes 20 either in groups of comb-like structures or individually onto a common base 1 (Fig. 1). The conductive area on each probe 20 may comprise the electrodes 5 and connector pads 8 on the connector part 3. The cavity 4 may have an electrically insulating inner surface. This can be an electrically insulating layer deposited on the substrate, e.g. semiconductor substrate, in which the cavities are produced (e.g. a SiO₂ layer deposited on Si), or said substrate 1 can itself be made from an insulating material (e.g. a polymer). Overhanging conductive, e.g. metal, blades 7 are present at the side of the cavities, to establish electrical contact between the probes 2 and the base 1 (see further). In another application, microstructures or probes 20 with fluidic channels can be inserted in cavities 4 in the base 1.

This allows direct drug delivery during electrophysiological monitoring. Another possibility is to locally isolate clusters of neurons to measure neuron activity on cell level.

The etched cavity with the overhanging conductive, e.g. metal, blades 7 can also be a MEMS connector which can be integrated with fluidics. This MEMS connector can be used in applications such as adaptive lens configuration and piezoelectric or acousto-electric sensing. Introducing the MEMS connector with optics and fluidics would be very interesting for opto-fluidic applications.

The MEMS connector can also be used when integrating different techniques on a medical catheter like chemical- and biological sensors. Integration of optical actuators and sensors can be very useful for biomedical image processing and pattern recognition.

In general, the probe 20 can comprise a sensing and/or actuating part at the top, a shaft 2 comprising conductor parts or electrodes 5, 8, and a connector part 3 at the bottom for being inserted in cavities 4 of the base 1. Electrical connections and other connections, such as microfluidic channels can be included on/in the probe 20, running over/in the shaft down to the connector part 3 at the bottom.

An example of a probe 20 can be a needle for sensing and actuating purposes in the brain. It can comprise a chisel-type tip, a needle-like shaft 2, and a connector part 3 at the bottom. Each probe shaft 2 can contain a number of electrodes 5. In case of assembling passive probes 20 the number of electrodes 5 on the shaft 2 may be limited because the amount of contact pads on the connector part 3 of the probe 20 is limited. A pitch density of 70 µm up to 35 µm can be achieved on the connector part 3. In that case the number of electrodes 5 can vary between 1 and 50 or between 1 and 25, or between 5 and 11. In case of assembling active probes 20, so when multiplexing is done, the shaft 2 of the probe 20 can be completely covered with electrodes 5. In the latter case, for example CMOS processing can be used to realise the high density of electrodes 5.

Probes 20 can be mounted on a base 1 individually or comb-like in groups to facilitate assembly. Also pairs or multiple combs of probes 20 can be assembled on a common connector part 3. Also probes 20 with additional functionality such as microfluidics to dispense drugs and/or extract body fluids as well as biosensors to detect biomolecules can be added.

The probes 20 can be mounted substantially orthogonally on the base 1 (angle = 90° or a slight deviation thereof, e.g. less than 10%, preferably less than 5%, more preferred less than 1%, still more preferred less than 0.1%) or can make a predetermined angle with the base 1. The angle between the probes 20 and the base 1 can be between 90° and 80°, between 90° and 70°, between 90° and 60°, between 90° and 45°, between 80° and 70°, between 85° and 60°, between 89° and 45°.In principle, any other angle different from zero can also be achieved to mount the probes under a predetermined angle with respect to the base 1.

The connector part 3 of a probe 20 is adapted to be inserted in the base 1. Therefore, cavities or holes 4 are provided in the base 1 to mount the different kinds of probes 20, depending on the needs of the application. The base 1 can comprise an array of several cavities 4. This can be for example 10 x 10 cavities, 5 x 5 cavities, or 4 x 4 cavities, 2 x 4 cavities, 5 x 10 cavities or any other combination that may be required for a particular application. The pitch between cavities 4 can vary, but is not limited hereto, between 100 µm and 2000 µm , between 200 µm and 1000 µm, or between 300 µm and 700 µm.

The in-plane dimensions of the cavity 4 can match the dimensions of the connector part 3 of the microstructures or probes 20, in such a way that it allows inserting the connector part 3 of the probe 20. The in-plane dimensions (x and y) can be, but are not limited hereto, in between 100 µm and 2000 µm , even better between 200 µm and 500 µm, or even better between 300 µm and 400 µm. The in-plane dimensions x and y can be the same or can be different, i.e. a cross-section of the cavity 4 in a direction parallel to the substrate surface can have a square or a rectangular shape, respectively. However, in alternative embodiments the cross-section of the cavity 4 in a direction parallel to the substrate surface can have any other suitable shape, such as e.g. circular, oval, trapezoidal, etc.

According to embodiments of the invention, to easily insert the connector parts 3 of the probes 20 into the base 1, the connector parts 3 should have a shape corresponding to the shape of the cavities 4, and the in-plane dimensions of the cavities 4 may substantially match the dimensions of the connector part 3 in at least one direction. With substantially match is meant that the cavities 4 and the connector parts 3 of the probes 20 may have substantially the same dimensions, although the dimensions of the connector parts 3 can be a little smaller that the dimensions of the cavities 4 in order to allow easy insertion of the connector parts 3 into the cavities 4. To mechanically clamp the microstructures or probes 20 in the cavities (for example by optimising the dimensions, geometry, and materials of the overhanging blades), the difference in dimensions can thus be chosen relatively small. The difference in dimensions between the in-plane dimensions of the connector part 3 and the cavities or holes 4 in the base 1 can be, but is not limited hereto, between 1 µm and 10 µm, even better between 3 µm and 8 µm, even better between 4 µm and 6 µm.

According to other embodiments, the cavities 4 and the connector parts 3 of the probes 20 may have different shapes and the in-plane dimensions of the cavities 4 may substantially match the dimensions of the connector parts 3 in one direction. For example, the cavities 4 may have a square shape while the connector parts 3 have a rectangular shape. The length of the rectangular connector parts 3 may be of substantially a same size as the sides of the square cavities 4. The width of the rectangular connector parts 3 may then be smaller than the size of the sides of the square cavities 4. In that way, the in-plane dimensions of the cavities 4 may substantially match the dimensions of the connector parts 3 only in one direction.

After assembly, the inserted microstructure 20 can make contact with interconnects for making, for example electrical contact. This can be achieved in different ways. According to an embodiment, the overhanging blades 7 can clamp or press the inserted microstructure 20 in the holes or cavities 4 (see Fig. 2).

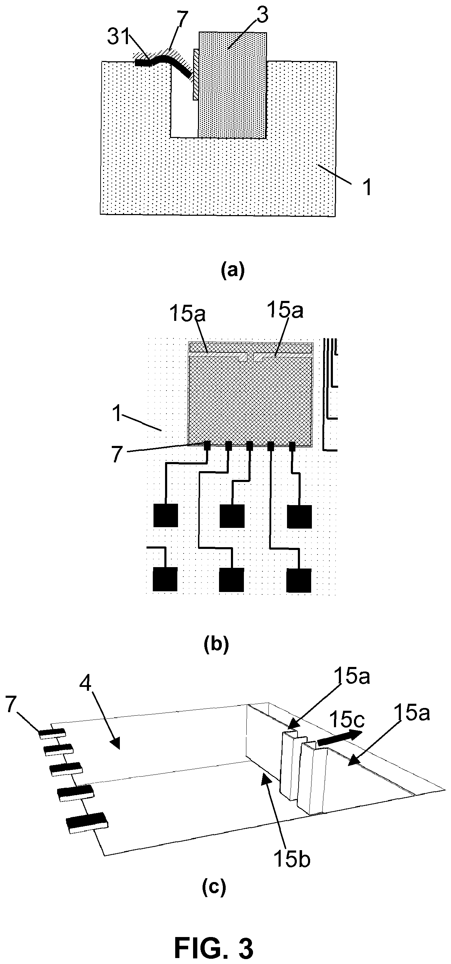

In another embodiment, clamping and electrical connection can be realised by using an elastic material 31, for example silicone, underneath the overhanging blades 7, e.g. metal blades (see Fig. 3 (a)). Also, the difference in dimensions between the connector part 3 and the cavity 4 can be cancelled out. In that way, an elastic blade 7, 31 may be obtained. The elastic blade 7, 31 (which may then comprise an elastic material 31 coated with blade material e.g. gold) tends to go back to its original form and will be pressed against a contact pad in the assembled structure..

In another embodiment, a built-in clamping structure 15 a can be used after assembly to press the inserted structure against the interconnects. This is illustrated in Fig. 3 (b) and (c). Fig. 3 (b) shows a top view and Fig. 3 (c) shows a three-dimensional view of a clamping system according to embodiments of the present invention. One side of the cavity 4 may comprise overhanging blades, e.g. gold blades and another side may comprise the clamping structure 15a which may, according to the present example, comprise elastically movable or thus flexible beams, e.g. silicon beams 15a. The elastically movable or thus flexible beams, e.g. silicon beams 15a, are attached with one side to a sidewall of the cavity 4. In between the bottom of the cavity 4 and the clamping structure 15a there may exist a gap which is in Fig. 3 (c) indicated by reference number 15b. Hence, it can be said that the clamping structure 15a, e.g. the silicon beams 15a are movably attached to the cavity 4. When the microstructure 20 is assembled into the cavity 4 the clamping structure 15a, in the example given the silicon beams 15a, which may also be referred to as cantilevers, are pressed backwards, i.e. in a direction away from the overhanging blades 7, e.g. gold blades (indicated by arrow 15c in Fig. 3 (c)), thereby offering resistance to and thus exerting a counter force on the connector part 3 of the microstructure 20 in a direction towards the overhanging blades 7, e.g. gold blades. In that way, the connector part 3 of the microstructure 20 may be clamped in the cavity 4.

In another embodiment, glue dispensing inside the cavity 4 prior to assembly can enhance the mechanical stability and can press the microstructure against the contacts.

In another embodiment, the dimensions of the connector part 3 can be slightly smaller or even a little larger than the dimensions of the cavities 4. The difference in dimension is preferable less than one micrometer. In case the dimensions of the connector part 3 are slightly larger than the dimensions of the cavities 4, to insert and mechanically clamp or lock the microstructures or probes 20 in the cavities 4, according to this embodiment, the base 1 can be heated, such that it expands and consequently the cavities 4 become slightly larger Expanding of the cavities 4 occurs because the depth of the cavity 4 is much smaller then the thickness of the base 2 (For 8 inch wafers this will be around 725 µm). The temperatures that can be used depend on the material of the base 1, on the properties of the material or materials deposited on the base 1, and on the material properties of the inserted microstructure 20. The temperature can be for example between 50°C and 500°C, between 100°C and 300°C, between 150°C and 250°C, but is not limited hereto. Depending on the bulk material of the base 1 the cavity 4 will expand thereby realising larger dimensions of the cavities 4 due to the thermal expansion coefficient and the probe or microstructure 20 can be inserted in the cavities 4. After cooling down, the dimensions of the cavities 4 go back to their original size and the probe 20 is clamped into the cavity 4. If clamping is too limited, extra clamping means can be used, for example by any of the embodiments described above.

In another embodiment, another way of inserting the microstructures 20 in the cavities 3 in case the dimensions of the connector part 3 are a little larger than the dimensions of the cavities 4, is cooling of the inserted microstructures or probes 20. For example cooling by using liquid nitrogen can realise a bigger temperature difference between the base 1 and the probes 20. The dimensions of the probes 20 will shrink depending on the material of the probes 20 and consequently the probes 20 can be assembled in the cavities 4 of the base 1. Going back to room temperature, the probes 20 return to their original size and are then clamped in the cavities 4. In that case, the overhanging blades 7 can for example be bent and squeezed between the cavity wall and the contact pad 8 of the probe or microstructure 20 resulting in a mechanical stable assembly. If needed, also extra clamping can be used, for example by any of the embodiments described above.

The electrical connection can also be made by a metal caulking technique Metal caulking is a way of thermo-compressive bonding of metal. In this technique, pressure is increased locally such that locally a higher temperature is obtained such that the metal can locally melt so as to form an electrical connection or bond. The assembly can be done using a flip chip bonder.

The aspect ratio (depth/width) of the cavities or holes can be between 0.2 and 1, even better between 0.3 and 0.7, even better between 0.4 and 0.6.

The depth of the cavities or holes can be several hundreds of µm. The depth can vary between 50 µm and 2000 µm, even better between 100 µm and 1000 µm, or even better between 150 µm and 250 µm. In some embodiments the cavities or holes are not etched completely through the wafer.

When the depth of the cavities 4 is limited, i.e. when the depth of the cavities 4 is much smaller than the thickness of the base 1, the thickness of the base 1, for example a silicon wafer, can be reduced, for example by grinding it. This may be done before or after formation of the cavities 4. Thinning of the base 1 results in a thinner base in which the microstructures or probes 20 can be inserted. This can be interesting for example in neural applications, where it could allow the base 1 with probes 20 to float with the brain i.e. not attached to the skull. The thickness of the base 1 can be between 150 µm and 2500 µm , for example between 50 µm and 2000 µm, between 200 µm and 1200 µm, between 100 µm and 1000 µm, or between 200 µm and 400 µm , for example between 150 µm and 250 µm. In case of, for example, an 8 inch silicon wafer the initial thickness is 725 µm, which can be thinned down to a thickness of, for example, between 100 µm and 600 µm, between 200 µm and 500 µm or between 250 µm and 400 µm

A small notch 6 in the cavity 4 (see Fig. 1) can facilitate alignment of the connector part 3 with respect to the base 1. The connector part 3 then has a corresponding slot. The size of the notch 6, and hence of the slot, can vary and may for example be, but is not limited hereto, between 10 µm and 500 µm , between 20 µm and 200 µm, or between 30 µm and 100 µm.

Provisions can be made to stop insertion of the probe 20 at a certain depth in the cavities 4. For this purposes, the notch 6 mentioned above can also be used. For example, the notch 6 can have a protrusion which blocks the probe 20 from being inserted deeper. In alternative embodiments, the height of the notch may be larger than the height of the corresponding slot in the probe 20, so as to also stop insertion of the probe 20 at a predetermined dept, being the dept of the slot. Stopping the probes 20 at a certain depth can also be done by adding a small piece inside the cavity 4 at a predetermined depth. This could also be done by a movable piece, such that the depth of the probe 20 inside the cavity 4 can be changed. The movable piece may, for example, be a plate which fits in the cavity 4 and which may form a movable bottom side of the cavity 4. By moving the plate, the depth of the cavity 4 can be varied. The cavities 4 can also be shaped such that they have a width becoming smaller and smaller when going deeper in the cavity 4. The cavities 4 then have a tapered shape. In that way, this can also stop the probes 20 at a predetermined depth, depending on the sizes of the cavities 4 and of the probes 20.

To stop the microstructures and/or probes 20 at a certain depth in the cavity 4, also the connector part 3 of the microstructure 20 can be made wider at a certain distance above the bottom of the connector part 3. This may be done step-wise or by using a connector part 3 having, for example, a conical shape. The distance above the bottom of the connector part 3 defines the depth at which the connector part 3 can be inserted into the cavities 4. Stopping the probes 20 at a certain depth can also be achieved when probes 20 are mounted in groups (see Fig. 1). In that case, connecting parts 3 of neighbouring probes 20 may be connected to each other by connecting bridges 12. Those connecting parts 12 between different microstructures or probes 20 also allow inserting the probes 20 until a certain depth into the cavities 4.

On the connector part 3 of the microstructure 20 there can be conductive lines 13 or conductive areas electrically connected to the individual sensors at the top of the probes 20 and electrodes 5 on the shaft (see Fig. 1). In-plane to off-plane electrical connection can be made between the microstructures 20 and the base 1. Microstructures 20 which are assembled orthogonally or under an angle to the base 1 can be electrically connected to the base 1. The microstructures 1 can be designed such that only in-plane electrically conductive, e.g. metal, lines and/or areas on both the probe 20 and the base 1 are used. This is advantageous because in plane electrically conductive, e.g. metal, lines and areas can easily be prepared with standard deposition and patterning techniques, in contrast to 3-dimensional contacting structures. Thereby the device allows making electrical contact between the in-plane electrically conductive, e.g. metal, lines/areas on the probe 20 and in-plane electrically conductive, e.g. metal, lines/areas/flaps on the base 1.

Electrical connectivity between the electrodes 5 on the microstructure shaft 2 and electrodes on the base 1 can be provided by building overhanging electrically conductive blades or flaps 7 on the edge of the cavities 4 in the base 1 in which the microstructures 20 are introduced. These blades 7 are matching with electrically conductive areas on the connector part of the microstructures 20.

The overhanging contact blades 7 fold into the assembly cavity upon insertion of the connector part 3 of the microstructures (Fig. 2). Once inserted, the blade 7 is squeezed between the sidewall of the cavity 4 and the microstructure 20. The electrodes and/or conductive areas and/or conductive lines on the connector part 3 of the microstructure 20 can match the overhanging blades on the base 7. The locations of the conductive areas on the connector part 3 can be chosen such that there is electrical contact between these conductive areas and the blades 7 on the base 1, when bent into the cavity 4 after insertion of the microstructure 20. Insertion allows electrical connectivity between two perpendicular contact pads. In case of a small difference in dimensions between the probes 20 and the cavities 4, electrical connection can be achieved between electrically conductive, e.g. metal, blades 7 on the base 1 and the electrically conductive, e.g. metal, lines on the probes 20. In that case electrical connection can, for example, be realised by metal caulking.

The present structure allows making a plurality of connections per microstructure 20, as illustrated in Fig. 4. It enables multiple, high density interconnects between parts. Fig. 4(a) shows a top view of the cavity 4, which depicts a plurality of overhanging connecting blades 7 which enable multiple electrical connections per microstructure 20. The lines depict metal tracks that end as overhanging blades 7 on the cavity 4. Connecting blades 7 can be extensions of conventional in-line electrically conductive, e.g. metal, tracks on the base 1 and are therefore compatible with various interconnecting schemes such as e.g. wire bonding.

Fig. 4(b) depicts the connector part 3 of a microstructure 20 inserted in a cavity 4. The picture shows in-line electrically conductive, e.g. metal, tracks or electrodes 5 and/or connector pads 8 on the microstructure 20 which match the connecting blades 7 (horizontal lines) on the base 1.

When the dimensions, i.e. shape and sizes, of the connector part 3, the cavity 4 and the overhanging blades 7 are well-chosen, locking of the microstructure 20 in the base 1 can be obtained by the overhanging blades 7, by thermal shrinkage of the connector part 3, and/or by thermal expansion of the cavity 4. The electrical connection can be made by pressing the electrical connections of the probes 20 against the conductive blades 7 on the base 1, by caulking or by any other method used in the field.

The width of the overhanging blades 7 can be between 1 µm and 100 µm, between 2 µm and 100 µm, between 5 µm and 50 µm, or between 10 µm and 30 µm and the length of the overhanging blades 7 can be between 1 µm and 100 µm, between 2 µm and 100 µm, between 3 µm and 50 µm, or between 5 µm and 20 µm. The thickness of the overhanging blades 7 can be in between 0.5 µm and 10 µm, for example between 1 µm and 5 µm or between 1.5 µm and 2 µm.

The pitch between blades 7 depends on the number of electrodes 5 on the microstructure 20. For instance, a five-electrode probe arrangement can use a 70 µm pitch between overhanging blades 7. The interconnect density can be increased by using smaller pitches, for example between 100 µm and 20 µm , between 80 µm and 30 µm or between 70 µm and 35 µm.

Changing the elastic and/or mechanical properties of the blades 7 can allow improved clamping the microstructure or probe 20 in the base 1, and may improve the electrical contact between the electrodes 5 on the microstructure or probe 20 and the base 1. Also using another, for example elastic material, underneath the blades 7 can facilitate clamping of the microstructure or probe 20 in the base 1.

The blades 7 can have different shapes or geometries. This results in different mechanical and elastic properties. Fig. 5 shows different implementations for the conductive blade 7. The blades 7 may have different shapes. For example, they can be straight lines (Fig. 5(a)), for example to relieve the stress imposed on the blade during insertion. The blades 7 can also bend slightly inside the cavity 5 (Fig. 5(b)) for example to provide additional spring action to ensure that the blade 7 is actually pushing against the matching conductive strip on the microstructure 20. Or the blades 7 can have a wave-like shape (Fig. 5(c)) for example to facilitate mechanical clamping of the microstructure 20 into the cavity 4. To increase the stiffness of the blade 7 during bending, the blade geometry and/or blade cross section can be changed. For example, T-, I- or U-profiles (in cross-section) may have different stiffness properties.

For changing the elasticity or mechanical properties of the overhanging blade 7, different conductive materials and/or different metals, compounds of different metals and/or conductive materials, stacks of different metals and/or conductive materials, or stacks of metals and other conductive and non-conductive materials (with a conductive material at the top) can be used. Examples are Au/Sn alloys and Au/ln alloys. To provide higher elastic properties the conductive material can be deposited on polymeric materials, for example silicone, BCB (Benzocyclobutene) and/or polyimide.

The blades 7 on the base 1 and the conductive areas on the probes 20 can be made of any conductive material. This can be a semiconductor, a conductor, a metal, such as Cu, Au, Ag, Al, or any other material that is used in the field.

These conductive regions can also be made by CMOS processing techniques, including deposition and patterning. Therefore it can be interesting to chose conductive materials that can be processed with CMOS techniques, for example Cu, Al, ...

To facilitate bending of the blades 7 in the cavities 4, the blades 7 can be made of a flexible conductive material, such as gold (Au), copper (Cu), or Indium (In).

The conductive material of the blades 7 and the conductive areas of the probe 20 can be the same material or can be a different material. In the best case, the conductive materials of the blades 7 and of the probes 20 are chosen such that they realise good electrical connection.

Particular materials allow improving the electrical contact between conductors on the connector part 3 of the microstructure or probe 20 and the blades 7 upon heating the microstructure 20. An example is a Au/ln alloy, whereby Indium changes shape and makes good electrical contact upon heating.

The blades 7 and/or conductive areas can be made of a single material or of different materials. In case of different materials a layered structure can be used. In case only one material is used, only conductive materials can be used. In case different materials are used to form the blades 7, a combination of conductive and non-conductive materials (for example flexible materials for clamping purposes) can be used.

The electrical contact between a blade 7 on the base 1 and the contacting pad 8 on the out-of-plane structure or probe 20 can be improved by depositing a solder material on the blade 7. After assembling the out-of-plane structure 20 into the base 1, a low temperature annealing can be applied. In this way the solder will reflow and an intermetallic compound is formed between the blade 7 and the contact pad 8. Different solder materials can be used such as In and Sn.

The microstructure 20 and the base 1 can, in addition to the blades 7, also be secured together for example by gluing, bonding, welding or by means of a built-in clamping or locking mechanism . An example of such a built-in clamping mechanism is represented in Fig. 3 (b) and (c). In that case, as already discussed above, the built-in clamping mechanism may comprise two flexible beams, e.g. silicon beams 15a, which are movably attached to a sidewall of the cavity 4. The flexible beams, e.g. silicon beams 15a, can be fabricated such that they bend into the cavity 4 in between the connector part 3 and the sidewalls of the cavity 4 upon insertion of the probe 20. When the dimensions and the material properties of the beams 15a are well-chosen, the connector part 3 may be fixed inside the cavity 4. Electrical and microfluidic connectivity between the matching features can be provided, while maintaining mechanical integrity.

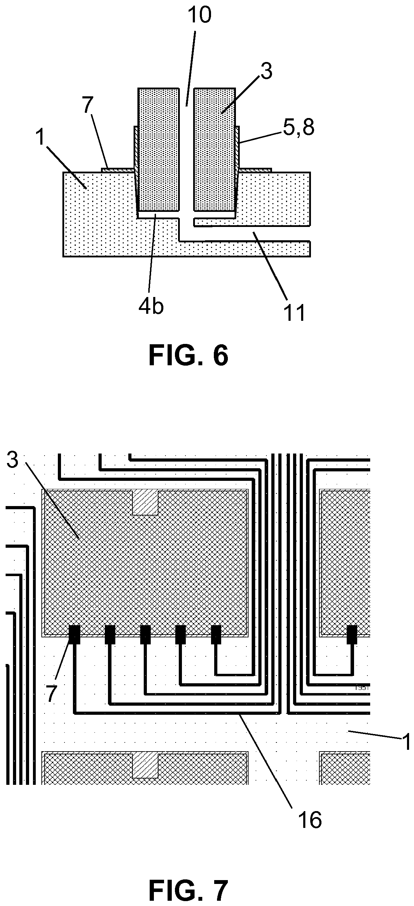

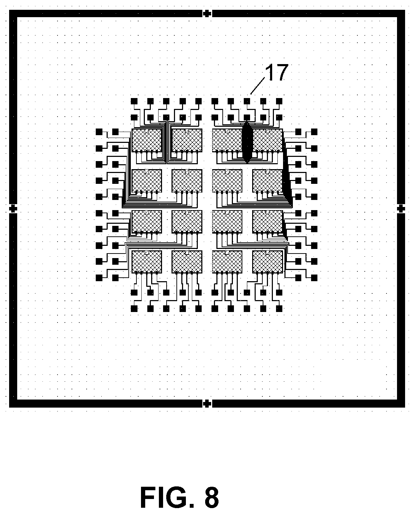

The conductive blades 7 on the base 1 can be connected to other circuitry via conductive lines 16 on the base or the substrate 1 (see Fig. 7). The conductive blades 7 on the base 1 can for example be connected to bond pads, conductive strips or integrated circuitry or CMOS circuitry on or in the base or substrate 1. The overhanging conductive blades 7 on the base 1 can also be electrically connected to external circuitry. To facilitate the connection to external circuitry, larger electrically conductive, e.g. metal, blades 1 or bond pads 17 can be added on the base, that are electrically connected with the electrically conductive, e.g. metal, blades 7 and lines on the base 1, for example by using conductive lines. An example of such wiring is illustrated in Fig. 8. The connection between the base 1 and external circuitry can be done for example using highly flexible ribbon cables for example polyimide or silicone. Any other method to connect the electrically conductive, e.g. metal, lines or blades 7 on the base 1 to other equipment can be used.

To avoid short circuits between the different electrodes, the probe 20 and/or base 1 can be made out of insulating material. Examples are silicon with low conductivity, ceramics, glass, SU8-photoresist, PDMS (polydimethylsiloxane) or other polymers. The probe 20 and/or base 1 can also be made of any other material provided that it is covered with an insulating material and thus which comprises at least a surface of an insulating material, on which the electrodes are created. For example, the probe 20 and/or base 1 can be made of semiconductor material, e.g. silicon, covered with a silicon dioxide layer. The microstructure 20 can also be coated with a biocompatible coating such as parylene, polyimide or BCB or any other biocompatible material.

The electrodes and/or conductive areas and/or interconnecting tracks and/or conductive lines on the probe 20 and base 1 can be made of any conductive material. For the conductive material a metal can be used such as platinum, gold, aluminum, copper, titanium, gold, or copper. For biocompatibility, titanium, platinum and gold can be used. Also other biocompatible materials can be used.

For some applications microfluidic channels can be included in probes 20 and in the base 1 (see Fig. 6). Microfluidic channels 10 in the probe 20 arrive in the connector part 3 of the probe 20. These can then be connected to microfluidic channels 11 in the base 1, terminating at one side in the cavities 4 in the base 1. The channels 11 in the cavities 4 can be located such that they are contacting the channel(s) 10 in the connector part 3 of the probe 20. This is schematically shown in Fig. 6, which depicts a cross-section view of a connector part 3 of a probe 20 assembled in a base 1, including microfluidic channels 10, 11 respectively in the probe 20 and in the base 1. To process microfluidic channels 10, 11, for example in semiconductor material such as e.g. silicon, different fabrication technologies can be used, for example bulk micromachining or surface micromachining. When the microfluidic channels 11, 12 are processed in plastics, injection moulding, hot embossing, casting techniques and lamination techniques can be used.

From Fig. 6, it can be observed that, once assembled, there can still be a part 4b of the cavity 4 left between the base 1 and the probe 20. So the microfluidic channels 10, 11 of both the probe 20 and the base 1 can lead to the cavity 4b between them - which can be sealed by the securing process - thereby forming a single microfluidic conduit running between the two connecting microstructures. Sealing between the two parts should allow electrical connections and connecting microfluidics. Methods for the sealing of the junction between the probe 20 and the backbone 1 include (but are not limited to) using a glass frit or a previously deposited layer (on the probe 20 as well as on the backbone 1) of low melting point glass (such as different types of spin-on glass); once the probes 20 are inserted, the whole structure is heated up and the glass layers are bonded to each other.

At the other side of the base 1, the microfluidic channels 11 in the base 1 can terminate at measurement and/or fluid-control equipment. This can allow the transport of fluids from the probes 20, through the base 1, towards other equipment, as well as in the opposite direction.

These microfluidic channels 10, 11 can be used for drug delivery, i.e. the administration of drugs, for example in case of cerebral applications.

Also other types of probes 20 can be designed to match the common backbone 1. As the different probes 20 and the backbone 1 can be designed independently, diverse probe configurations can be generated, thereby extending the versatility of the resulting probe arrays.

The probes 20 can also comprise functional regions connected via conductive areas or wires with the conductive blades 7. That way conductive strips, wires or areas on the connector part 3 of the microstructure or probe 20 can be electrically connected to functional regions on the microstructure or probe 20. These functional regions on the microstructure or probe 20 allow different measurements and can also allow activation. When the microstructure or probe 20 is inserted in a cavity 4, the overhanging conductive blades or flaps 7 bend inside the cavity 4 and are contacting the conductive strips on the connector part 3 of the microstructure or probe 20.

The base 1 with cavities or holes 4 should at least have an insulating surface on which the electrically conductive, e.g. metal, blades 7 and electrodes can be created. According to embodiments, the base 1 may be formed of an insulating material such as e.g. a polymer. According to other embodiments, the base 1 may be formed of a semiconductor material such as silicon having an insulating layer on top. Thereby, for example biocompatible materials can be used to form an insulating layer. The base 1 can be made using moulds in which a polymer is introduced. Thereby LIGA (X-ray lithography) can be used. SU8-photoresists or PDMS can be used as a material to be introduced in the mould. The base 1 can also be made in a silicon wafer using CMOS processing techniques. Also other methods for making the base 1 can be used.

Below a fabrication method for the base 1 is described, in the example given starting from a semiconductor substrate such as e.g. a Si substrate. It has to be understood that this is only by way of an example and that this is not intended to limit the invention in any way. Fig. 9 in its sub-sections shows subsequent steps of the exemplary fabrication process. First, a base 1 with cavities or holes 4 can be created. Afterwards, the overhanging electrically conductive, e.g. metal, blades 7 can be fabricated. The overhanging blades 7 can be fabricated using a process based on planarization.

A semiconductor substrate 1, for example a silicon wafer (Fig. 9(a)) can be coated with a layer 21 (Fig. 9(b)) that can be used as an etch mask for etching the cavities or holes 4. On silicon, for example PECVD (Plasma Enhanced Chemical Vapour Deposition) silicon dioxide can be used. This layer can be patterned, for example by RIE (reactive ion etch) to serve as an etch mask for etching the cavities or holes 4 in the silicon wafer. This last can be done by DRIE (deep reactive ion etch) (Fig. 9(c)). The wafer 1 provided with cavities 4 can then be coated with an insulating material 22, for example a PECVD oxide layer (Fig. 9(d)).

To start the fabrication of the overhanging blades 7, the substrate or wafer 1 can be planarized with a sacrificial material 24 at the side of the cavities 4. The sacrificial material 24, which may also be referred to as planarizing material, can be chosen such that there is already a high degree of planarization after depositing the sacrificial material 24. To limit problems during further processing, outgassing of the sacrificial material 24 as such can be limited. To fill an etched cavity 4, that can be several hundred microns deep, a thick viscous material can be used. If the sacrificial material 24 does not fill the cavities 4 completely, there can be holes in the sacrificial material 24 containing gasses that might be outgassing during further processing: this may cause problems during further processing. So in the best case, the material 24 can fill the cavities 4 completely. If needed, the planarizing material 24 can be cured. Examples of planarizing sacrificial materials 24 are polyimide (PI2525, DuPont), BCB (XU35075 Dow Company) as well as certain spin-on-glasses. The sacrificial material 24 can be deposited over the entire surface (Fig. 9(e)). The sacrificial material 24 may be provided for example by spin coating. In order to easily remove the sacrificial material 24 later in the process flow, in embodiments where an imidized polymer is used as the sacrificial material 24, it may be advantageous if the polymer is not completely imidized.

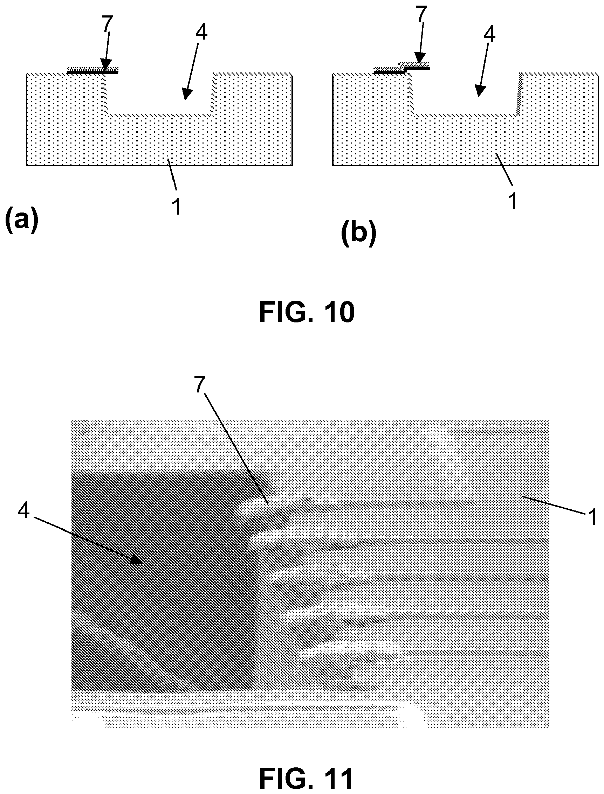

For some applications bending of the blade 7 may be needed as illustrated in Fig. 10(b). Therefore the excess sacrificial material 24 above the wafer surface may be reduced to a thickness of ∼5 µm using a planarizing process (Fig. 9(f)), for example thinning the surface with a wafer grinder (DFG8560, Disco Corp.) or Fly cutter (DFS8910, Disco Corp.). A lithography step can be performed to define a region slightly bigger than the original cavity 4 (in the process step represented in Fig. 9(g)), e.g. by providing a mask 30 and then removing the material not covered by the mask 30,. The purpose of this is to create a small step 25. The step 25 will be transferred to any blade 7 made on this step. This step 25 in the blade 7 will facilitate the folding of the blade 7 into the cavity 4 and will avoid the blade 7 from breaking while it is pressed against the sharp edge of the cavity 4. Subsequently, the planarizing material (for example polyimide or BCB) in between the cavities 4 is removed (Fig. 9(g)). This can be done by, for example, plasma etching, wet etching or reactive ion etching. But any other suitable method to etch/remove this material known by a person skilled in the art can be used. As a consequence, the planarizing material 24 (for example polyimide) remains in the areas slightly larger than the cavities, i.e. protected in the previous step,

If the sharp edge of the cavity 4 doesn't cause problems during bending of the blade 7, the blade 7 can be straight (see Fig. 10(a)). In that case, the excess of planarizing material 24 is completely removed in Fig. 9(e) and (f), such that there is no step 25 above the etched cavities or holes, in contrast to Fig. 9(g).

Subsequently, the overhanging blades 7 and connecting lines and conductive areas can be fabricated. This can be done by deposition and patterning of electrically conductive material, e.g. metal. In one approach, electrically conductive material, e.g. metal, is deposited all over the surface, followed by lithography and dry and/or wet etching. In another approach, patterns are defined in for example a resist layer 26 defining the locations of the electrically conductive, e.g. metal, tracks and overhanging blades or flaps 7. This is followed by the deposition of an electrically conductive, e.g. metal, layer on the entire surface. Then the excess electrically conductive layer, e.g. metal, on top of the resist is removed by a lift-off technique. Electrically conductive, e.g. metal, tracks remain on the regions not protected by the resist.

Overhanging blades 7 and connecting lines and conductive areas can also be fabricated by plating. An example is given in case of gold, but any other material that can be plated can be used. A seed layer 29, for example TiW/Au/TiW or Ti/Au/Ti can be deposited, by for example sputtering (Fig. 9(h)). This is followed by another lithography step, which defines the regions where metallic (for example gold) interconnects and the overhanging blades 7 will be created. A resist layer 26 is patterned, thereby creating holes 27 in the resist layer 26 at the locations where the electrically conductive blades 7, connecting lines and conductive areas are to be created (Fig. 9(i)). An electrically conductive, e.g. metallic, layer 28, for example gold, can then be deposited, using for example electroplating (Fig. 9(j)). This will be plated on the regions not protected by the patterned resist layer 26. Afterwards, the resist layer 26 can be removed (Fig. 9(k)) as well as the remaining seed layer (Fig. 9(I)), thereby leaving the overhanging blades 7 and interconnects. Finally, the sacrificial planarizing layer 24 (for example polyimide) can be completely removed from the cavities 4, with, in case of polyimide, for example a NaOH or KOH base solution, Microstrip 2001 (Olin Microelectronic Materials), or EKC265 which results in the structures shown in Fig. 9(m). In case BCB is used as sacrificial layer, Primary stripper A (Dow Company) can be used. That way overhanging blades 7 on cavities 4 can be created.

The present invention can, for example, be used to make a device for recording single neuron electrical activity in the brain. The device comprises an array of probes 20, for example needles. These probes 20 may have different functionalities. The probes 20 can comprise a plurality of electrodes 5 or other biological applications or microfluidic channels 10, 11. These probes 20 can be inserted in cavities 4 in a base 1, the cavities 4 comprising overhanging blades 7 that are bent into the cavities 4 to ensure electrical contact to the electrodes 5 on the probes 20. The base 1 can be made thin which is advantageous when placed in the skull. The modular approach allows choosing probe shape, size and functionality suited for applications involving complex brain regions; it enables the integration of electrical, microfluidic and biosensor probes within the same base 1. Biosensors can be integrated on the probe shafts 2. They can be used for in-vivo monitoring of chemical substances in the brain. That way exploration of fundamental processes and the interaction of chemical and electrical information transfer in the brain can be assessed. Other biological applications such as neuronal stimulation in the brain, peripheral nerve recording and stimulation, biochemical probing of organs such as the liver, are also envisaged.

Once the base is obtained, for example through the above process, probes 20 or comb-like groups of probes 20 can be inserted into the cavities 4. In this embodiment, the probes 20 can be secured to the cavity 4 by using adhesive such as medical-grade cyanoacrylate adhesive or locally-applied spin-on glass.

### Experiment

A probe 20 was made of silicon and comprises a chisel-type tip, a needle-like silicon shaft 2, and a connector part 3 at the bottom (see Fig. 1). Each probe 20 had a sharp tip with an angle of 17°. The length of the combined tip and silicon shaft varied from 2 to 9 mm. The shaft 2 had a width of 120 µm and a thickness of 100 µm. The dimensions of the base of the probe were 297 µm by 395 µm (corresponding to the dimensions of the cavity being 300 µm by 400 µm). Combs with 4 probes 20 were made (see Fig. 1), that could be inserted into a base 1. On each probe shaft 2 there were a number of equidistantly distributed electrodes 5. The number of electrodes 5 varied between 5 up to 9 electrodes 5 per probe 20. The electrodes 5 were connected to metal contacts at the bottom of the probe 20, which were matching the overhanging connecting blades 7 on the base 1.

In the silicon backbone 1 there was an array of 4 x 4 cavities 4 covered by silicon oxide. The size of each cavity 4 was 300 µm x 400 µm , with a small notch of 50 µm x 50 µm to facilitate alignment. The pitch between cavities was 550 µm x 550 µm. The backbone 1 was made out of silicon covered with a SiO₂ layer.

For electrical connectivity, overhanging conductive Au blades 7 on the edge of the cavities 4 were made. They were located such that they were matching the conductive blades 7 with conductive strips on the connector part 3 of the probe 20 (see Figs. 2(a), 11, and 12). The overhanging blades 7 were made of gold and had a thickness between 2 µm and 4 µm. The overhanging blades 7 are 20 µm wide and their overhanging parts were in between 5 µm to 20 µm long. The pitch between blades 7 depended on the number of electrodes 5 envisaged for the probe 20. In case of a five-electrode probe arrangement, a 70 µm pitch between overhanging blades 7 was used.

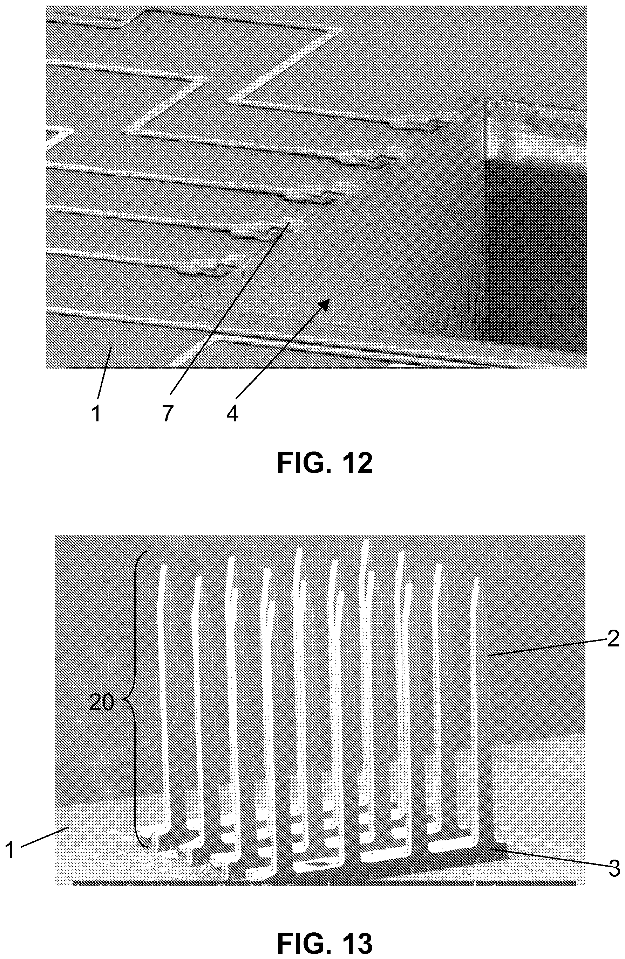

Upon insertion (Figs. 2(b), (c), (d)), such blades 7 were pushed into the cavity 4 in the base 1 and were then squeezed between the side wall of the cavity 4 and the conductive strips of the connector part 3, thereby establishing electrical contact. This is illustrated in Fig. 2 (d) (lateral cross-section). The probes 20 were mechanically secured to the cavity 4. Fig. 13 shows a comb-like group of probes 20 assembled on the backbone 1.

The electrical interconnection of the electrodes 5 to an external circuitry was obtained via conductive wires or interconnecting tracks on the common backbone 1. Therefore the overhanging contact blades 7 were connected to conductive wires or interconnecting tracks on the backbone 1 (Figs. 7, 8, 11, and 12). These interconnecting tracks on the base were made of gold.

The fabrication process of the base or support 1 is represented in Fig. 9. A silicon wafer 1 (Fig. 9(a)) was coated with PECVD silicon dioxide (Fig. 9(b)), which was patterned to serve as the etch mask for the next step, which consists of a DRIE (deep reactive ion etch) of silicon to obtain the cavities (Fig. 9(c)) where probes 20 will be inserted. After this, a second PECVD oxide layer was deposited (Fig. 9(d)). At this stage the wafer was planarized in order to start the fabrication of the overhanging blades 7. A thick layer 24 of BCB (XU35075, Dow company) is applied over the entire surface and soft baked (Fig. 9(e)). The excess BCB material above the wafer surface was reduced to a thickness of 5 µm using a wafer grinder (DFG8560, Disco Corp.) (see Fig. 9(f)). A lithography step was performed to define a region slightly bigger than the original cavity 4. The remaining BCB (except for the areas protected in the previous step, which include the cavities) was then removed using reactive ion etching (Fig. 9(g)). A seed layer comprising of TiW/Au/TiW was then deposited by sputtering (Fig. 9(h)). This was followed by another lithography step (Fig. 9(i)), which defines the regions where gold interconnects (including the overhanging blades 7) will be created. Figs. 11 and 12 show details of a group of blades 7 at this step. Gold was then deposited through electroplating (Fig. 9(j)) and the resist (Fig. 9(k)) and remaining seed layer were removed (Fig. 9(I)). Finally the BCB was completely removed from the cavities 4 using Primary stripper A (Dow company) (Fig. 9(m)). The resulting cavity 4 with overhanging Au blades 7 is shown in Figs. 11 and 12, i.e. a scanning electron micrograph of the cavity 4 at an angle, showing the overhanging connecting blades 7 on the cavity 4.

Assembly of the microstructures or probes 20 was done with a flip chip bonder (Suss FC150 automatic flip chip bonder). To enhance the assembly and to ensure mechanical clamping, the bottom chuck was heated till 250 °C while the tooling chuck, containing the microstructure or probes 20 was kept at room temperature. As a result the cavities 4 expanded due to the thermal expansion coefficient. After assembly the platform was cooled down. As a result the microstructure or probes 20 were fixed into the base 1.

It is to be understood that although preferred embodiments, specific constructions and configurations, as well as materials, have been discussed herein for devices according to the present invention, various changes or modifications in form and detail may be made without departing from the scope of this invention as defined by the appended claims.

## Claims

1. A device for sensing and/or actuating comprising:
a substrate (1) comprising at least one cavity (4) formed in its main surface, wherein said substrate (1) and said at least one cavity (4) have at least an insulating surface;
at least one microstructure (20) comprising a connector part (3) and at least one shaft (2), the connector (3) part of each microstructure (20) being inserted in one of said cavities (4) by means of an insertion in a direction orthogonal to the main surface of the substrate, said microstructure (20) comprising conductive lines (5,8) extending partially on said connector part (3) and partially on said shaft (2), which connector part fits into and matches said cavity as a male and a female part;
a series of flexible conductive blades (7) at the at least one cavity (4), comprising a first part and a second part, the first part of the at least one flexible conductive blade (7) being outside said cavity (4) and present on the substrate and the second part leading over the cavity (4), the second part being located and squeezed between a sidewall of said cavity (4) and said connector part (3) such that a conductive blade (7) is in one to one electrical contact with a conductive line on said connector part (3).

2. The device as claimed in claim 1, wherein the flexible conductive blades comprise an elastic material coated with blade material.

3. The device as claimed in claim 1, wherein the cavity comprises a notch for facilitating alignment between the microstructures and the cavity, the connector part (3) having a corresponding slot.

4. The device as claimed in claim 1, wherein the cavity further comprises a built-in clamping structure to press the microstructure against the conductive blades.

5. The device as claimed in claim 4, wherein the clamping structure comprises elastically movable and thus flexible beams, that are attached with one side to a sidewall of the cavity (4).

6. The device as claimed in claim 5, wherein the elastically movable beams are silicon cantilever beams.

7. The device according to any of the previous claims, further comprising first microfluidic channels (11) in said substrate (1) and second microfluidic channels (10) in said at least one microstructure (20), said first and second microfluidic channels (11, 10) being connected to each other with sealed holes in said cavities (4).

8. The device according to any of the previous claims, wherein said at least one microstructure (20) is a needle.

9. The device according to any of the previous claims, wherein the flexible blades (7) comprises a step facilitating folding of the blades (7) into the cavity (4) and avoiding breaking of the blades (7) while pressed against an edge of the cavity (4).

10. The device according to any of the previous claims, wherein said substrate (1), said at least one microstructure (20), said series of conductive blades (7), and conductive lines are made of or covered with biocompatible materials.

11. A method for assembling on a substrate (1) microstructures (20) for sensing and/or actuating, said method comprising the steps of:
obtaining a substrate (1) with at least one cavity (4) formed in its main surface, the substrate (1) and the at least one cavity (4) having an insulating surface, on which substrate (1) a series of flexible conductive blade (7) is present near each cavity (4), said conductive blades (7) partially overhanging said at least one cavity (4)
obtaining at least one microstructure (20) comprising a connector part (3), at least one shaft (2), and conductive strips extending partially on said connector part (3) and partially on said shaft (2), said connector part (3) being shaped such as to fit into and matching with the at least one cavity (4), said conductive strips on said connector part (3) being located such as to contact said conductive blades (7) near said at least one cavity (4) upon insertion;
inserting said connector part (3) of said at least one microstructure (20) in said at least one cavity (4) by means of an insertion in a direction orthogonal to the main surface of the substrate, thereby bending said series of flexible conductive blades (7) into said at least one cavity (4) so as to end up squeezed between a sidewall of the at least one cavity (4) and the microstructure and realizing one to one electrical contact between said flexible conductive blades (7) and said conductive strips on said connector parts (3) of said at least one microstructure; and
fixing said connector parts (3) inside said at least one cavity (4).

12. A method as claimed in claim 11, wherein providing said series of flexible conductive blades (7) comprises the steps of
filling said at least one cavity (4) with a sacrificial material (24);
providing the series of flexible conductive blades (7) partially on said sacrificial material (24);
optionally providing conductive paths in electrical contact with said conductive blades (7);
removing said sacrificial material (24) from said at least one cavity (4).

13. The method as claimed in claim 12, wherein after provision of the sacrificial material a lithography step is performed to define a region slightly bigger than the original at least one cavity (4), so as to create a step, that is subsequently transferred to the blades upon their provision

14. The method as claimed in any of claims 11 to 13, wherein the dimensions of said connector part (3) are slightly larger or smaller than the dimensions of said at least one cavity (4) and fixing said connector parts (3) in said at least one cavity (4) comprises the steps of
creating a temperature difference between said substrate (1) and said microstructure (20) such as to allow insertion of the connector parts (3) into the at least one cavity (4);
inserting the connector parts (3) in the at least one cavity (4); and
bringing said substrate (1) and said microstructure (20) at the same temperature.

15. Use of a device according to any of claims 1-10 for measurements and/or actuation of neural activity.

## Patentansprüche

1. Vorrichtung zum Erfassen und/oder Betätigen, umfassend:
ein Substrat (1), das mindestens einen Hohlraum (4) umfasst, der in seiner Hauptfläche ausgebildet ist, wobei das Substrat (1) und der mindestens eine Hohlraum (4) mindestens eine Isolierfläche aufweisen;
mindestens eine Mikrostruktur (20), die einen Verbindungselementteil (3) und mindestens einen Schaft (2) umfasst, wobei der Verbindungselementteil (3) jeder Mikrostruktur (20) mittels einer Einführung in einer Richtung orthogonal zu der Hauptfläche des Substrats in einen der Hohlräume (4) eingeführt wird, wobei die Mikrostruktur (20) Leiterbahnen (5, 8) umfasst, die sich teilweise auf dem Verbindungselementteil (3) und teilweise auf dem Schaft (2) erstrecken, wobei - als aufgenommenes und aufnehmendes Teil - der Verbindungselementteil in den Hohlraum hineinpasst und diesem entspricht;
eine Reihe von flexiblen leitfähigen Lamellen (7) an dem mindestens einen Hohlraum (4), die einen ersten Teil und einen zweiten Teil umfassen, wobei der erste Teil der mindestens einen flexiblen leitfähigen Lamelle (7) sich außerhalb des Hohlraums (4) befindet und auf dem Substrat vorliegt und der zweite Teil über den Hohlraum (4) hinweg führt, wobei der zweite Teil (sich) derart zwischen einer Seitenwand des Hohlraums (4) und dem Verbindungselementteil (3) befindet und eingezwängt ist, dass eine leitfähige Lamelle (7) in elektrischem Eins-zu-Eins-Kontakt mit einer Leiterbahn auf dem Verbindungselementteil (3) steht.

2. Vorrichtung nach Anspruch 1, wobei die flexiblen leitfähigen Lamellen ein elastisches Material umfassen, das mit Lamellenmaterial beschichtet ist.

3. Vorrichtung nach Anspruch 1, wobei der Hohlraum eine Kerbe zum Erleichtern einer Ausrichtung zwischen den Mikrostrukturen und dem Hohlraum umfasst, wobei der Verbindungselementteil (3) einen entsprechenden Schlitz aufweist.

4. Vorrichtung nach Anspruch 1, wobei der Hohlraum ferner eine eingebaute Klemmstruktur umfasst, um die Mikrostruktur gegen die leitfähigen Lamellen zu drücken.

5. Vorrichtung nach Anspruch 4, wobei die Klemmstruktur elastisch bewegliche und somit flexible Träger umfasst, die mit einer Seite an einer Seitenwand des Hohlraums (4) befestigt sind.

6. Vorrichtung nach Anspruch 5, wobei die elastisch beweglichen Träger Kragträger aus Silicium sind.

7. Vorrichtung nach einem der vorhergehenden Ansprüche, die ferner erste Mikrofluidkanäle (11) in dem Substrat (1) und zweite Mikrofluidkanäle (10) in der mindestens einen Mikrostruktur (20) umfasst, wobei die ersten und zweiten Mikrofluidkanäle (11, 10) in den Hohlräumen (4) über abgedichtete Löcher miteinander verbunden sind.

8. Vorrichtung nach einem der vorhergehenden Ansprüche, wobei die mindestens eine Mikrostruktur (20) eine Nadel ist.

9. Vorrichtung nach einem der vorhergehenden Ansprüche, wobei die flexiblen Lamellen (7) einen Absatz umfassen, der ein Hineinfalten der Lamellen (7) in den Hohlraum (4) erleichtert und ein Brechen der Lamellen (7), während sie gegen einen Rand des Hohlraums (4) gedrückt werden, vermeidet.

10. Vorrichtung nach einem der vorhergehenden Ansprüche, wobei das Substrat (1), die mindestens eine Mikrostruktur (20), die Reihe von leitfähigen Lamellen (7) und die Leiterbahnen aus biokompatiblen Materialien hergestellt oder mit solchen bedeckt sind.

11. Verfahren zum Zusammenbauen von Mikrostrukturen (20) zum Erfassen und/oder Betätigen auf einem Substrat (1), wobei das Verfahren folgende Schritte umfasst:
Erhalten eines Substrats (1) mit mindestens einem Hohlraum (4), der in seiner Hauptfläche ausgebildet ist, wobei das Substrat (1) und der mindestens eine Hohlraum (4) eine Isolierfläche aufweisen, wobei auf dem Substrat (1), nahe jedem Hohlraum (4), eine Reihe von flexiblen leitfähigen Lamellen (7) vorhanden ist, wobei die leitfähigen Lamellen (7) teilweise über den mindestens einen Hohlraum (4) überstehen;
Erhalten mindestens einer Mikrostruktur (20), die einen Verbindungselementteil (3), mindestens einen Schaft (2) und Leiterbahnen umfasst, die sich teilweise auf dem Verbindungselementteil (3) und teilweise auf dem Schaft (2) erstrecken, wobei der Verbindungselementteil (3) derart geformt ist, dass er in den mindestens einen Hohlraum (4) hineinpasst und diesem entspricht, wobei die Leiterbahnen auf dem Verbindungselementteil (3) derart angeordnet sind, dass sie, bei einer Einführung, nahe dem mindestens einen Hohlraum (4) mit den leitfähigen Lamellen (7) in Kontakt gelangen;
Einführen des Verbindungselementteils (3) der mindestens einen Mikrostruktur (20) in den mindestens einen Hohlraum (4) mittels einer Einführung in einer Richtung orthogonal zur Hauptfläche des Substrats und dadurch Biegen der Reihe von flexiblen leitfähigen Lamellen (7) in den mindestens einen Hohlraum (4) hinein, so dass sie schließlich zwischen einer Seitenwand des mindestens einen Hohlraums (4) und der Mikrostruktur eingezwängt sind, und Realisieren eines elektrischen Eins-zu-Eins-Kontakts zwischen den flexiblen leitfähigen Lamellen (7) und den Leiterbahnen auf den Verbindungselementteilen (3) der mindestens einen Mikrostruktur; und
Befestigen der Verbindungselementteile (3) im Inneren des mindestens einen Hohlraums (4).

12. Verfahren nach Anspruch 11, wobei ein Bereitstellen der Reihe von flexiblen leitfähigen Lamellen (7) folgende Schritte umfasst:
Füllen des mindestens einen Hohlraums (4) mit einem Opfermaterial (24);
Bereitstellen der Reihe von flexiblen leitfähigen Lamellen (7) teilweise auf dem Opfermaterial (24);
optional Bereitstellen von Leiterbahnen in elektrischem Kontakt mit den leitfähigen Lamellen (7);
Entfernen des Opfermaterials (24) aus dem mindestens einen Hohlraum (4).

13. Verfahren nach Anspruch 12, wobei nach der Bereitstellung des Opfermaterials ein Lithographieschritt durchgeführt wird, um einen Bereich zu definieren, der etwas größer ist als der ursprüngliche mindestens eine Hohlraum (4), um einen Absatz zu schaffen, der nachfolgend - im Zuge ihrer Bereitstellung - auf die Lamellen übertragen wird.

14. Verfahren nach einem der Ansprüche 11 bis 13, wobei die Abmessungen des Verbindungselementteils (3) etwas größer oder kleiner sind als die Abmessungen des mindestens einen Hohlraums (4) und das Befestigen der Verbindungselementteile (3) in dem mindestens einen Hohlraum (4) folgende Schritte umfasst:
Erzeugen einer Temperaturdifferenz zwischen dem Substrat (1) und der Mikrostruktur (20) derart, dass eine Einführung der Verbindungselementteile (3) in den mindestens einen Hohlraum (4) möglich ist;
Einführen der Verbindungselementteile (3) in den mindestens einen Hohlraum (4); und
Bringen des Substrats (1) und der Mikrostruktur (20) auf die gleiche Temperatur.

15. Benutzung einer Vorrichtung nach einem der Ansprüche 1-10 für Messungen und/oder eine Betätigung der Nervenaktivität.

## Revendications

1. Dispositif de détection et d'activation comprenant :
un substrat (1) comprenant au moins une cavité (4) formée dans sa principale surface, ledit substrat (1) et ladite au moins une cavité (4) ayant au moins une surface isolante ;
au moins une microstructure (20) comprenant une partie connecteur (3) et au moins un arbre (2), la partie connecteur (3) de chaque microstructure (20) étant insérée dans l'une desdites cavités (4) par le biais d'une insertion dans une direction orthogonale par rapport à la surface principale du substrat, ladite microstructure (20) comprenant des lignes conductrices (5, 8) se prolongeant partiellement sur ladite partie connecteur (3) et partiellement sur ledit arbre (2), laquelle partie connecteur s'encastre dans et correspond à ladite cavité en tant que partie mâle et partie femelle ;
une série de lames conductrices souples (7) au niveau de l'au moins une cavité (4), comprenant une première partie et une seconde partie, la première partie de l'au moins une lame conductrice souple (7) étant à l'extérieur de ladite cavité (4) et présente sur le substrat et la seconde partie avançant sur la cavité (4), la seconde partie étant située et coincée entre une paroi latérale de ladite cavité (4) et ladite partie connecteur (3) de telle sorte qu'une lame conductrice (7) est en contact électrique en tête à tête avec une ligne conductrice sur ladite partie connecteur (3).

2. Dispositif selon la revendication 1, dans lequel les lames conductrices souples comprennent un matériau élastique revêtu d'un matériau de type lame.

3. Dispositif selon la revendication 1, dans lequel la cavité comprend une encoche pour faciliter l'alignement entre les microstructures et la cavité, la partie connecteur (3) ayant une fente correspondante.

4. Dispositif selon la revendication 1, dans lequel la cavité comprend en outre une structure de serrage intégrée pour presser la microstructure contre les lames conductrices.

5. Dispositif selon la revendication 4, dans lequel la structure de serrage comprend des poutres élastiquement déplaçables et donc souples, qui sont attachées à un côté d'une paroi latérale de la cavité (4).

6. Dispositif selon la revendication 5, dans lequel les poutres élastiquement déplaçables sont des poutres en porte-à-faux en silicium.

7. Dispositif selon l'une quelconque des revendications précédentes, comprenant en outre des premiers canaux microfluidiques (11) dans ledit substrat (1) et des seconds canaux microfluidiques (10) dans ladite au moins une microstructure (20), lesdits premiers et seconds canaux microfluidiques (11, 10) étant raccordés les uns aux autres par des trous étanches dans lesdites cavités (4).

8. Dispositif selon l'une quelconque des revendications précédentes, dans lequel ladite au moins une microstructure (20) est une aiguille.

9. Dispositif selon l'une quelconque des revendications précédentes, dans lequel les lames souples (7) comprennent une étape facilitant le pliage des lames (7) dans la cavité (4) et évitant de briser les lames (7) lorsqu'elles sont pressées contre un bord de la cavité (4).

10. Dispositif selon l'une quelconque des revendications précédentes, dans lequel ledit substrat (1), ladite au moins une microstructure (20), ladite série de lames conductrices (7) et les lignes conductrices sont constitués de ou revêtus de matériaux biocompatibles.

11. Procédé d'assemblage sur un substrat (1) des microstructures (20) pour la détection et/ou l'activation, ledit procédé comprenant les étapes consistant à :
obtenir un substrat (1) ayant au moins une cavité (4) formée dans sa surface principale, le substrat (1) et l'au moins une cavité (4) ayant une surface isolante, sur ledit substrat (1), une série de lames conductrices souples (7) est présente à proximité de chaque cavité (4), lesdites lames conductrices (7) formant saillie sur ladite au moins une cavité (4)
obtenir au moins une microstructure (20) comprenant une partie connecteur (3), au moins un arbre (2) et des bandes conductrices se prolongeant partiellement sur ladite partie connecteur (3) et partiellement sur ledit arbre (2), ladite partie connecteur (3) étant façonnée de telle sorte qu'elle s'encastre dans et correspond à ladite au moins une cavité (4), lesdites bandes conductrices sur ladite partie connecteur (3) étant situées de sorte à être en contact avec lesdites lames conductrices (7) à proximité de ladite au moins une cavité (4) lors de l'insertion ;
insérer ladite partie connecteur (3) de ladite au moins une microstructure (20) dans ladite au moins une cavité (4) par le biais d'une insertion dans une direction orthogonale par rapport à la surface principale du substrat, pliant ainsi ladite série de lames conductrices souples (7) à l'intérieur de ladite au moins une cavité (4) de sorte qu'elles finissent écrasées entre une paroi latérale de l'au moins une cavité (4) et la microstructure et réaliser un contact électrique en tête à tête ente lesdites lames conductrices souples (7) et lesdites bandes conductrices sur lesdites parties connecteurs (3) de ladite au moins une microstructure ; et
fixer lesdites parties connecteurs (3) à l'intérieur de ladite au moins une cavité (4).

12. Procédé selon la revendication 11, dans lequel le fait de fournir ladite série de lames conductrices souples (7) comprend les étapes consistant à remplir ladite au moine une cavité (4) avec un matériau sacrificiel (24) ; utiliser la série de lames conductrices souples (7) partiellement sur ledit matériau sacrificiel (24) ;
utiliser facultativement des voies conductrices en contact électrique avec lesdites lames conductrices (7) ;
retirer ledit matériau sacrificiel (24) de ladite au moins une cavité (4).

13. Procédé selon la revendication 12, dans lequel après l'utilisation du matériau sacrificiel, une étape de lithographie est réalisée pour définir une région légèrement plus grande que l'au moins une cavité (4) initiale, de sorte à créer une différence de niveau, qui est ensuite transférée sur les lames lors de leur utilisation.

14. Procédé selon l'une quelconque des revendications 11 à 13, dans lequel les dimensions de ladite partie connecteur (3) sont légèrement plus grandes ou plus petites que les dimensions de ladite au moins une cavité (4) et la fixation desdites parties connecteurs (3) dans ladite au moins une cavité (4) comprend les étapes consistant à
créer une différence de température entre ledit substrat (1) et ladite microstructure (20) de sorte à permettre une insertion des parties connecteurs (3) à l'intérieur de l'au moins une cavité (4) ;
insérer les parties connecteurs (3) à l'intérieur de l'au moins une cavité (4) ; et amener ledit substrat (1) et ladite microstructure (20) à la même température.

15. Utilisation d'un dispositif selon l'une quelconque des revendications 1 à 10 pour la mesure et/ou l'activation de l'activité nerveuse.
